# FASCICULE DE BREVET EUROPEEN

(11) **EP 1 647 540 B1**
(45) Date de publication et mention de la délivrance du brevet: **19.03.2008**
(21) Numéro de dépôt: 05292030.3
(22) Date de dépôt: 29.09.2005
(51) Int. Cl.: C07C 29/76, C13D 3/08

(54) **Procede de preparation de l-iditol**
Verfahren zur Herstellung von L-Idit
Process for the preparation of L-iditol

(30) Priorité: 15.10.2004 FR 0410973
(43) Date de publication de la demande: 19.04.2006
(73) Titulaire: ROQUETTE FRERES, 62136 Lestrem (FR)
(72) Inventeur: Fuertes, Patrick, 59160 Lomme (FR)
(74) Mandataire: Boulinguiez, Didier

(56) Documents cités:
- EP-A- 1 388 530
- US-A- 4 422 881
- M. OGAWA, ET AL.: "Microbial production of optically pure L-iditol by yeast strains" APPLIED AND ENVIRONMENTAL MICROBIOLOGY, vol. 46, no. 4, octobre 1983 (1983-10), pages 912-916, XP008047444 AMERICAN SOCIETY FOR MICROBIOLOGY, WASHINGTON, DC, US ISSN: 0099-2240
- V. VONGSUVANLERT, ET AL.: "L-Iditol production from L-sorbose by a methanol yeast, Candida boidinii (Kloeckera sp.) No. 2201" JOURNAL OF FERMENTATION TECHNOLOGY, vol. 66, no. 5, 1988, pages 517-523, XP002331358 TOKYO, JP
- E.I. FULMER, ET AL.: "The effect of the concentration of sorbitol upon the production of sorbose by the action of Acetobacter suboxydans" JOURNAL OF THE AMERICAN CHEMICAL SOCIETY, vol. 58, no. 6, juin 1936 (1936-06), pages 1012-1013, XP002331486 AMERICAN CHEMICAL SOCIETY, WASHINGTON, DC, US
- Y. KHOUVINE: "Réduction del'alpha-d-glucoheptulose par le nickel de Raney" COMPTES RENDUS HEBDOMADAIRES DES SEANCES DE L'ACADEMIE DES SCIENCES, vol. 204, 1937, pages 983-984, XP002331485 GAUTHIER-VILLARS, MONTREUIL, FR

## Description

L'invention a pour objet un procédé de préparation de L-iditol de haute pureté.

Le L-iditol est un hexitol de saveur sucrée qui pourrait trouver des applications dans le domaine alimentaire s'il advenait qu'on puisse le produire en grandes quantités et à bas prix ou s'avérer être un intermédiaire de synthèse pour la préparation d'autres produits et notamment de produits d'anhydrisation interne tels que l'iditan et surtout l'isoidide ou 1,4-3,6-dianhydroiditol.

Ces anhydrides dont les groupements hydroxyle et notamment les deux groupements -(OH) de l'isoidide s'adonnent très facilement à des réactions d'éthérification ou d'estérification deviennent à leur tour des intermédiaires intéressants quoiqu'ils puissent trouver aussi en eux-mêmes, des applications comme agents osmotiques ou rétenteurs d'eau notamment.

L'estérification de l'isoidide avec des acides monocarboxyliques permet d'obtenir des diesters, employables comme plastifiants dans des résines de synthèse. Les di-éthers, tels que le diméthylisoidide, s'avèrent d'excellents solvants pour certains actifs pharmaceutiques ou encore pour certains polymères naturels ou de synthèse.

L'isoidide peut en outre constituer en lui-même l'un des motifs monomériques de polyesters, en liaison avec des diacides et d'autres alcools bivalents.

Par analogie avec l'hydrogénation du D-glucose qui fournit du D-sorbitol avec un rendement presque total, le L-iditol pourrait être obtenu avec un rendement quasi-stoechiométrique par hydrogénation du L-idose. Le L-idose n'est cependant pas un glucide que l'on trouve à l'état naturel et son extraction à partir de sources végétales par exemple, n'est donc pas envisageable.

Le L-iditol, anciennement appelé sorbiéritol, existe en faible quantité dans le jus de sorbes (fruits du sorbier), à côté de petites quantités de sorbitol (D-glucitol). Là encore, l'extraction à partir de ces baies n'est pas industriellement envisageable.

On sait que l'hydrogénation catalytique des cétoses conduit à l'obtention de deux stéréo-isomères. C'est ainsi que l'hydrogénation du L-sorbose permet d'obtenir en mélange, du D-sorbitol et son épimère en C5, le L-iditol.

L'utilisation de certains catalyseurs et/ou l'emploi de conditions particulières d'hydrogénation permettent de faire varier dans certaines limites les proportions des deux énantiomères. Le document " Diastereoselectivity control in ketose hydrogenations with supported copper and nickel catalysts " de J.F. RUDDLESDEN et coll. publié en 1981 indique par exemple qu'un catalyseur à base de cuivre permet d'obtenir, en hydrogénant le L-sorbose, 68% de D-glucitol (D-sorbitol) et qu'un catalyseur à base de nickel permet d'obtenir une majorité de l'autre énantiomère, à savoir 64% de L-iditol.

L'isomérisation des hexitols facilement disponibles tels que le sorbitol ou le mannitol permet également de former, quoique en quantité minoritaire de l'iditol. Le document " Catalytic isomerisation of the hexitols ; D-glucitol, D-mannitol, L-iditol and galactitol "de L. WRIGHT et L. HARTMAN, Journal of Organic Chemistry, Vol 26, Mai 1961 indique qu'à 170°C et sous une pression d'hydrogène de 1900 p.s.i. (130 bars) et en présence de catalyseur au nickel, on est en situation d'équilibre pour un système contenant 50% de sorbitol, 25% de mannitol et 25% d'iditol.

Ces deux procédés, hydrogénation des cétoses et isomérisation des hexitols, fournissent donc du D-iditol en quantités appréciables mais malheureusement avec une mauvaise pureté et un mauvais rendement dus à l'apparition ou à la persistance dans le milieu réactionnel d'autres polyols.

L'extraction du D-iditol à partir de ces mélanges réactionnels s'avère alors très complexe et elle est généralement effectuée en formant des composés d'addition chimique de ces divers polyols (aldéhyde benzoïque, anhydride acétique) et/ou des cristallisations en phase solvant organique, pyridine, alcool... de ces composés ou polyols.

Des procédés biologiques de réduction du L-sorbose de façon quasi-exclusive en L-iditol sont connus et permettent d'obtenir de très hautes richesses en L-iditol. Cependant ils n'ont jamais pu être employés industriellement, soit qu'ils fonctionnent avec de mauvais rendements, soit que leur mise en oeuvre s'avère impossible ou trop difficile à cette échelle.

Le document " Microbial production of optically pure L-iditol by yeast strains ", Appl. Environ. Microb. Vol. 46, n° 4, Pp 912-916, 1983 », enseigne que *Candida intermedia* permet d'obtenir 50 g/l de L-iditol à partir de 150 g/l de L-sorbose en 5 jours. Bien qu'il ne reste plus de L-sorbose dans le moût de culture en fin de fermentation, la cristallisation directe du L-iditol s'est révélée impossible, même après déminéralisation. Les auteurs ont dû passer par l'hexaacétate et la cristallisation dans le méthanol pour obtenir un des cristaux fondant entre 70 et 73° C.

Le document "L-iditol production from L-sorbose by a méthanol yeast, Candida boidinii (Kloeckera sp.) N°. 2201 ", J. Ferment. Technol., Vol 66, N°5,Pp 517-523, 1988, décrit un procédé purement enzymatique de réduction du L-sorbose en L-iditol en utilisant la D-sorbitol déshydrogénase et des cellules immobilisées de *C. boidinii* comme support de NADH dont la régénération est assurée par un ensemble d'enzymes d'oxydation du méthanol, ce méthanol constituant la source d'hydrogène assurant cette régénération. Le rendement de conversion s'établit à environ 96% en une quarantaine d'heures pour une concentration voisine de 150 g/l. Il n'est pas dit comment le L-iditol peut être isolé du milieu réactionnel. Ce procédé extrêmement complexe et délicat, n'a jamais été mis en oeuvre à l'échelle industrielle.

Par conséquent, on est toujours à la recherche d'un procédé propre à fournir abondamment du L-iditol avec un rendement et une pureté suffisamment élevés pour que l'industriel puisse disposer de cet hexitol en quantité abondante et dans des conditions avantageuses de prix.

C'est à ce problème posé depuis de fort nombreuses années que la Société Demanderesse a eu le mérite d'apporter une solution en trouvant qu'il était possible d'obtenir le L-iditol à l'état de haute pureté et dans des conditions économiques tout à fait avantageuses en procédant au fractionnement chromatographique d'un mélange de L-sorbose et de L-iditol sur résines ou zéolithes cationiques, les résines cationiques étant préférées et constituées par celles qui sont utilisées pour la séparation chromatographique des sucres tels que le glucose et le fructose ou des polyols tels que le sorbitol et le mannitol.

La Société Demanderesse a en effet remarqué au terme de nombreux essais que la séparation chromatographique du L-sorbose et du L-iditol s'effectuait beaucoup plus facilement que la séparation chromatographique du L-iditol et du D-sorbitol qui fournirait *a priori* un procédé beaucoup plus simple.

Les mélanges de L-sorbose et de L-iditol dont les premiers d'entre eux ont été obtenus par le chimiste français Gabriel BERTRAND au début du siècle dernier (G. Bertrand, Bull. Soc. Chim., 3° série, 1905, t. 33, p 166 et suiv. et p. 264-267) peuvent être obtenus comme indiqué par BERTRAND, par hydrogénation acide du L-sorbose puis oxydation bactérienne du mélange D-sorbitol / L-iditol obtenu par cette hydrogénation.

Ces mélanges peuvent aussi résulter d'une hydrogénation plus sélective du sorbose, orientant celle-ci vers une production accrue de L-iditol (J.F. RUDDLELDSEN) puis oxydation bactérienne de ce mélange.

Ils peuvent encore résulter d'un processus d'isomérisation des hexitols. Dans ce cas, la présence de mannitol se traduira, après l'oxydation bactérienne, par l'apparition correspondante de fructose qu'il conviendra de récupérer en même temps que le L-sorbose lors de l'étape de chromatographie afin de séparer ces deux cétoses du L-iditol.

Bien entendu, les différentes manières d'obtenir des mélanges contenant du L-iditol puis de les oxyder peuvent être combinées, l'invention se satisfaisant cependant mieux des mélanges qui ne contiennent pas ou qui ne contiennent que peu de mannitol et de fructose.

Avantageusement, les fractions chromatographiques rassemblant le ou les produits d'oxydation, à savoir essentiellement le L-sorbose mais aussi éventuellement du fructose, sont à nouveau hydrogénées, seules ou en présence de L-sorbose nouvellement mis en oeuvre. Ces fractions sont alors à nouveau soumises à l'oxydation bactérienne. Cette manière de faire permet d'obtenir quasi-exclusivement et avec un rendement stoechiométrique s'approchant de l'unité, le L-iditol à partir du L-sorbose.

On rappelle, ici que l'hydrogénation du fructose génère les deux hexitols épimères en C2 que sont le D-sorbitol et le D-mannitol et que dans le cas ou la fraction de sorbose issue de la chromatographie contient aussi du fructose, on retrouve après hydrogénation, une composition ternaire correspondant aux produits de l'isomérisation des hexitols et contenant D-sorbitol, D-mannitol et L-iditol.

On rappelle également ici, en termes actuels, la règle de BERTRAND que celui-ci avait établie en étudiant l'action de *Bacterium xylinum (Acetobacter)* sur toute une série de polyols et notamment sur ceux contenus dans les jus de sorbes : " La bactérie du sorbose réalise une déshydrogénation stéréospécifique portant sur la fonction alcool secondaire voisine de la fonction alcool primaire dans les cas ou les deux hydroxyles les plus proches de l'alcool primaire sont en position *cis* ". Cette bactérie, par les déshydrogénases à spectre large qu'elle synthétise, est ainsi capable d'oxyder le glycérol en dihydroxyacétone, l'érythritol en érythrulose, mais aussi le xylitol en xylulose, le sorbitol en sorbose et le mannitol en fructose, cette liste n'étant pas exhaustive.

Il s'ensuit que le procédé de préparation et de purification de L-iditol conforme à l'invention est caractérisé par le fait qu'un sirop d'un mélange de L-iditol et de L-sorbose est soumis à un traitement chromatographique conduisant à l'obtention d'au moins deux fractions dont l'une est fortement enrichie en L-iditol (fraction X1) et dont l'autre est fortement enrichie en L-sorbose (fraction X2).

De manière avantageuse, le traitement chromatographique est conduit de manière à ce que la fraction fortement enrichie en L-iditol (fraction X1) soit composée, les pourcentages étant exprimés en poids sur matières sèches:
- de 80 à 99,9%,
- de préférence, de 90 à 99,5% et, plus préférentiellement encore de 95 à 99,5% de L-iditol,
le complément à 100% étant constitué essentiellement par le L-sorbose.

Selon un mode de réalisation avantageux du procédé de l'invention, le mélange de L-iditol et de L-sorbose est obtenu par fermentation d'une solution de D-sorbitol et de L-iditol à l'aide d'un microorganisme producteur de déshydrogénases du genre *acetobacter* ou *gluconobacter,* propre à transformer le D-sorbitol en L-sorbose.

Selon un autre mode de réalisation avantageux du procédé de l'invention, la solution de D-sorbitol et de L-iditol soumise à fermentation est obtenue par hydrogénation catalytique de L-sorbose.

Selon un autre mode de réalisation plus particulièrement avantageux du procédé de l'invention, la fraction X2 fortement enrichie en L-sorbose est collectée pour être mélangée au L-sorbose destiné à l'hydrogénation catalytique.

Le L-sorbose est un cétose produit à l'échelle de plusieurs dizaines de milliers de tonnes par an dans le monde puisqu'il est un intermédiaire dans la synthèse de l'acide ascorbique ou vitamine C. Il est lui-même obtenu par fermentation d'une solution de D-sorbitol pur le plus souvent à l'aide des mêmes microorganismes qui sont employés pour fermenter les mélanges de D-sorbitol et de L-iditol dans le procédé de la présente invention.

Le D-sorbitol pur est produit dans le monde à l'échelle de plusieurs centaines de milliers de tonnes par an et il est obtenu par hydrogénation de solutions de D-glucose à haute richesse. Cette hydrogénation peut s'effectuer à l'aide des mêmes catalyseurs qui servent à hydrogéner les solutions de L-sorbose.

L'invention a donc aussi ceci de remarquable qu'elle permet d'utiliser des matériels communs: réacteurs et fermenteurs, aussi bien pour l'obtention du L-sorbose, que pour celle des mélanges de L-sorbose et de L-iditol à chromatographier. Les catalyseurs à mettre en oeuvre: métalliques pour l'hydrogénation d'une part et biologiques pour la fermentation d'autre part, sont également les mêmes. Il est donc particulièrement avantageux lorsque l'on met en oeuvre le procédé de l'invention, de regrouper les étapes menant du D-glucose au L-iditol sur un même site.

On consultera avec intérêt le schéma 1 joint qui reprend dans leur totalité les étapes menant du D-glucose au L-iditol, celles qui sont plus particulièrement revendiquées figurant en lettres et traits plus épais.

L'invention sera encore mieux comprise à l'aide du complément de description qui suit, de l'exemple illustratif mais non limitatif ainsi que des schémas ci-annexés, lesdits complément de description, exemple et schémas n'étant relatifs qu'à un mode de réalisation avantageux.

Dans le susdit schéma 1, les étapes M1 et M2 correspondent respectivement à l'hydrogénation du D-glucose en D-sorbitol puis à la transformation par voie fermentaire du D-sorbitol en L-sorbose. Ces étapes ne seront pas davantage détaillées puisqu'elles ont déjà suffisamment été décrites dans la littérature pour que quiconque du métier puisse les mettre en oeuvre sans difficultés. Elles pourraient de toute manière être mises en oeuvre dans les conditions décrites ci-dessous pour les étapes M3 et M4.

L'étape M3 correspond à l'hydrogénation du L-sorbose en un mélange de D-sorbitol et de L-iditol. Elle peut être menée dans les conditions décrites dans l'article de J.F. RUDDLESDEN et amenant un excès énantiomérique en L-iditol (64% voir table 5). Elle peut aussi être menée à l'aide d'un catalyseur au ruthénium ou au nickel de Raney, de manière continue ou discontinue. Lorsqu'on a recours à une hydrogénation discontinue, on préfère travailler en présence d'environ 5 à 10% de nickel de Raney avec une concentration en sorbose comprise entre 300 et 450 g de sucre par kilogramme de solution et à une température comprise entre 80 et 130° C. Pour éviter l'apparition de produits d'isomérisation des hexitols, on préfère maintenir le pH à une valeur inférieure à 8,0. La pression d'hydrogène est maintenue entre 20 et 80 bars jusqu'à ce que la teneur en sucres réducteurs du mélange hydrogéné devienne inférieure à 1% et de préférence à 0,1%. Suivent alors les sous-étapes classiques de purification de tout sirop hydrogéné, à savoir celles de la décantation puis de la filtration du catalyseur et celles de la purification des impuretés solubles par décoloration au noir animal ou sur charbon granulaire et déminéralisation sur résines échangeuses d'ions, cationique et anionique, fonctionnant respectivement dans le cycle hydrogène et hydroxyle.

L'étape M4 correspond à l'oxydation du mélange de D-sorbitol et de L-iditol causée par les déshydrogénases bactériennes. Lors de la fermentation correspondante, le sorbitol dont la fonction alcool secondaire située en C5 obéit à la loi de BERTRAND se voit oxydée en fonction cétone. Le D-sorbitol devient donc du L-sorbose. Le L-iditol dont aucune des quatre fonctions alcool secondaire ne remplit ces conditions, demeure inchangé au cours de la fermentation.

Pour la fermentation de ce mélange, on peut avoir recours à un milieu de culture présentant la composition suivante:

| | |
|---|---|
| Mélange de D-sorbitol et de L-iditol: | 100 à 200 g/l. |
| Azote organique (sous forme de corn-steep ou d'extrait de levure) | 2 g/l (extrait de levure). |
| KH2PO4 | 1 à 3 g/l. |
| MgSO4, 7H2O | 1 à 2 g/l. |

et qui est introduit dans un fermenteur, stérilisé puis inoculé à l'aide de 10% environ d'une préculture de 20 heures d'un microorganime du genre *Acetobacter* ou *Gluconobacter,* par exemple *Gluconobacter oxydans.*

La fermentation est poursuivie à une température de 25 à 35° C sous une aération correspondant à 1 à 1,5 volume d'air par volume de culture et par minute, à un pH de 4,0 à 6,0 et durant un temps généralement compris entre 24 et 48 heures, terme au bout duquel tout le D-sorbitol a été converti en L-sorbose.

Ce moût de fermentation peut alors être purifié de manière connue en elle-même par filtration ou centrifugation, décoloration au noir animal ou sur charbon granulaire puis déminéralisation sur résine cationique et anionique fonctionnant respectivement dans le cycle hydrogène et hydroxyle. Ce sirop purifié est ensuite concentré pour être soumis à l'étape de chromatographie M5.

Cette étape M5 de fractionnement chromatographique peut être effectuée de toute manière connue en elle-même, de façon discontinue ou continue ( SMB simulated moving bed, ISMB improved simulated moving bed, SSMB sequential simulated moving bed ...) sur des adsorbants du type résines cationiques fortement acides, de préférence chargées en ions alcalins ou alcalino-terreux ou encore du type zéolithes cationiques elles aussi chargées avec les mêmes ions.

Des exemples de tels procédés de séparation chromatographique sont donnés par exemple dans les brevets US 2.985.589, US 3.291.726, US 3.268.605 de la société U.O.P.

Suivant un mode de réalisation préféré, l'étape de séparation chromatographique est effectuée par mise en oeuvre du procédé et de l'appareillage décrits dans le brevet US 4.422.881 et son correspondant français FR 2.454.830 dont la Société Demanderesse est titulaire.

Quel que soit le procédé de séparation chromatographique retenu, on a recours, à titre d'adsorbant, à un matériau cationique, de préférence à une résine cationique forte, du type polystyrène sulfoné, cette résine étant plus préférentiellement encore employée sous forme ionique calcium et étant réticulée par le divinylbenzène à un taux d'environ 4 à 10%. Le choix des paramètres de l'étape de chromatographie, dont notamment:
- le débit d'élution,
- le débit d'alimentation en sirop du mélange L-sorbose / L-iditol,
- le débit d'extraction de la fraction X1 enrichie en L-iditol,
- la composition des zones de désorption, d'adsorption et d'enrichissement se trouve illustrée dans l'exemple.

Ce choix est fait de telle sorte que la fraction X1, fortement adsorbée par la résine, présente une richesse en L-iditol, les pourcentages étant exprimés en poids de matières sèches:
- de 80 à 99,9%,
- de préférence, de 90 à 99,5,% et, plus préférentiellement encore, de 95 à 99,5%,
le complément à 100% étant constitué essentiellement par le L-sorbose.

Pour aboutir à ces résultats, on choisit lesdits paramètres comme suit lorsque lorsque l'étape de chromatographie est effectuée par mise en oeuvre du procédé et de l'appareillage décrits dans le brevet US 4.422.811 et que l'adsorbant utilisé est une résine cationique de faible granulométrie, réticulée à 7% de divinylbenzène (PCR732 de la société PUROLITE) et utilisée sous forme calcium:
- débit d'élution de 375 à 450 l/h/m3 d'adsorbant,
- débit d'alimentation en sirop de mélange L-iditol / L-sorbose de 30 à 45 l/h/m3 d'adsorbant,
- débit d'extraction de la fraction enrichie en L-iditol de 100 à 140 l/h/m3 d'adsorbant.
L'étape de chromatographie conduit par ailleurs à l'obtention concomitante d'une fraction X2, exclue de la résine et fortement enrichie en L-sorbose.

Cette fraction X2 fortement enrichie en L-sorbose est de préférence recyclée, conformément à l'invention, à l'étape d'hydrogénation, commodément en mélange avec les moûts de fermentation issus de l'étape M2 de manière à profiter d'une purification commune. Ce recyclage est symbolisé par la lettre "R" figurant à droite de la flèche symbolisant celui-ci.

De préférence cette fraction X2 est composée, les pourcentages étant exprimés en poids sur matières sèches:
- de 60 à 99,5% de L-sorbose,
- de préférence, de 80 à 99% et plus préférentiellement encore, de 90 à 99% de L-sorbose,
le complément à 100% étant constitué essentiellement par le L-iditol.

La pureté de cette fraction X1 peut être telle qu'il est souvent possible de l'utiliser directement dans le domaine alimentaire ou comme matière première dans la fabrication de produits dérivés tels les produits de déshydratation interne que sont l'iditan ou l'isoidide. Cependant, cette fraction X1 qui constitue le L-iditol de haute pureté obtenu selon le procédé de la présente invention, se prête particulièrement bien à la cristallisation. Par simple concentration à une matière sèche voisine de 80%, de préférence suivie d'un refroidissement dans une cuve agitée, on obtient de façon massive de superbes cristaux que l'on sépare facilement de leurs eaux-mères par essorage. Il n'est nul besoin, pour procéder à cette cristallisation d'avoir recours à des solvants organiques. Cette manière de procéder permet d'augmenter la richesse du L-iditol bien au-delà de ce qu'elle était dans cette fraction X1.

### EXEMPLE:

On a soumis au fractionnement chromatographique, un mélange contenant 45% de L-sorbose et 55% de L-iditol. En réalité, ce mélange contient quelques traces d'autres sucres et polyols dans une teneur voisine de 2%. Ce mélange avait été obtenu en fermentant à l'aide de *Gluconobacter oxydans* ATCC 19357, un mélange de D-sorbitol et de L-iditol obtenu en hydrogénant une solution à 40% de L-sorbose. Cette hydrogénation effectuée à l'aide de nickel de RANEY dans des conditions de pH sensiblement neutre, avait fourni un sirop titrant 0,2% de sucres réducteurs résiduels et contenant D-sorbitol et L-iditol en quantités sensiblement équivalentes. Après la fermentation, ce sirop avait été purifié par filtration puis décoloration sur noir granulaire et enfin déminéralisation sur résines échangeuses d'ions avant d'être concentré à une matière sèche de 50%.

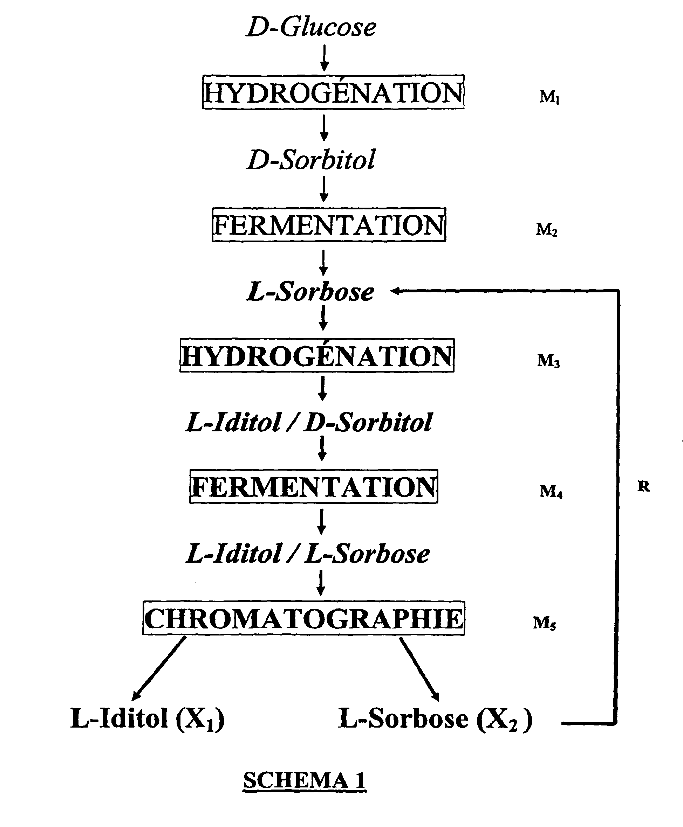

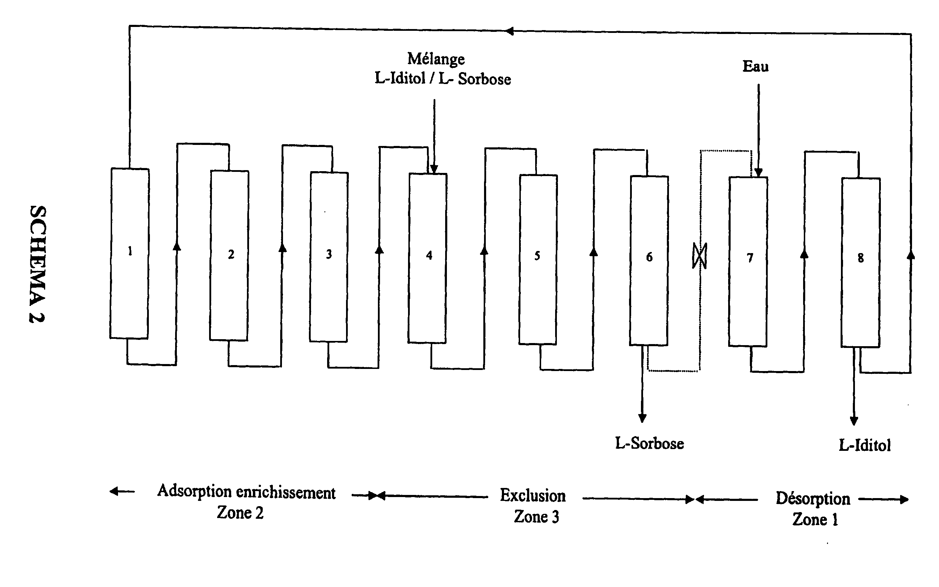

L'installation chromatograhique comprend, comme montré à la figure 2 du brevet américain US 4.422.881 dont le contenu est incorporé à la présente description par référence, huit colonnes ou étages de 200 litres chacune, garnies d'adsorbant du type résines cationiques fortes sous forme calcium et de granulométrie fine et homogène (0,2 à 0,3 mm) du type PCR732 de la société PUROLITE.

On règle dans cette installation, les ouvertures et fermetures des électrovannes créant le lit mobile simulé, de manière à établir une zone I de désorption du L-iditol de deux étages, une zone II d'adsorption-enrichissement de trois étages et une zone III d'exclusion du L-sorbose de trois étages comme montré sur le présent schéma 2 qui est une représentation simplifiée de la figure 2 du brevet américain US 4.422.881 et sur laquelle on n'a fait figurer que:
- les colonnes C1 à C8,
- le dispositif de fermeture assurant le sens du courant chromatographique, en l'occurrence l'électrovanne située entre les colonnes 6 et 7,
- la canalisation d'alimentation en sirop de L-iditol et de L-sorbose à séparer,
- la canalisation d'alimentation en eau d'élution assurant la séparation,
- la canalisation d'extraction de la fraction X1 enrichie en L-iditol figurant sur la colonne 8,
- la canalisation d'extraction de la fraction X2 enrichie en L-sorbose figurant sur la colonne 6,

Le dispositif de fermeture maintient dans la configuration adoptée, une étanchéité totale entre, d'une part, la zone III qui est une zone d'exclusion à l'extrémité de laquelle est récupéré le L-sorbose et d'autre part la zone I de désorption du L-iditol, zone en tête de laquelle est introduite l'eau de désorption.

Une minuterie assure pour une certaine durée et pour les débits indiqués ci-après, une alimentation suffisante en eau de désorption au premier étage ou première colonne de la zone I (colonne 7 sur le présent schéma 2), pour effectuer la désorption de la totalité du L-iditol ainsi qu'une alimentation (colonne 4) en un volume du mélange de L-iditol et de L-sorbose à séparer compatible avec le volume d'adsorbant contenu dans la colonne et sa capacité d'adsorption, de façon à obtenir un taux d'extraction du L-sorbose au moins égal à 80% du L-sorbose présent dans le mélange à séparer et cela, à une richesse au moins égale à 60% en sorbose.

Ces conditions étant remplies, la minuterie assure le décalage vers la droite sur la figure 2, d'un étage de l'ouverture et de la fermeture de toutes les électrovannes. Il faut donc assurer huit décalages ou cycles pour retrouver l'état représenté sur cette figure 2.

Les susdits taux d'extraction et puretés sont maintenus constants en ajustant le débit de la pompe d'extraction non montrée du L-iditol adsorbé (colonne 8). La sortie de la fraction L-sorbose exclu (colonne 6) s'effectue à pression atmosphérique et son débit constant résulte de la différence entre les débits d'alimentation et le débit d'extraction. Il est judicieux d'éliminer à chaque début de cycle la partie de cette fraction qui n'est constituée que par de l'eau.

Le mélange de L-iditol et de L-sorbose qui est introduit dans l'installation en tête de la zone III d'exclusion, présente, comme indiqué plus haut, une teneur en matières sèches voisine de 50%. La température à l'intérieur des colonnes de séparation est maintenue à environ 70° C. On a fait fonctionner l'installation dans les conditions suivantes:
- la minuterie est réglée pour une durée de cycle de 20 mn,
- le mélange de L-iditol et de L-sorbose à séparer est acheminé vers l'installation au débit de 60 litres/heure,
- l'eau assurant l'élution est acheminée vers l'installation au débit de 660 litres/heure,
- la fraction de L-iditol est extraite au débit de 198 litres/heure,
- la fraction de L-sorbose représentant le complément entre les débits des fluides entrant dans l'installation et ceux en sortant s'extrait donc spontanément au débit de 522 litres/heure. On ne conserve cependant que les dernières 10 minutes de chaque cycle, la période qui précède représentant une fraction ne contenant que de l'eau quasiment pure qui est récupérée pour l'élution.

Les tableaux 1 et 2 ci-dessous résument les conditions caractérisant le fonctionnement du dispositif chromatographique.

**TABLEAU 1**

| Entrées chromatographiques | Sirop L-Iditol et de L-Sorbose | Eau | Total |
|---|---|---|---|
| Débit l/h | 60 | 660 | 720 |
| Densité kg/l | 1,192 | 1,000 | - |
| Matières sèches | 50 % | 0 | 35,8 |
| Débit massique (kg/h) | 71,5 | 660 | 731,5 |
| Richesse en L-Iditol (% sec) | 55 | 0 | - |
| Débit massique en L-Iditol (kg/h) | 19,7 | 0 | 19,7 |
| Débit massique L-Sorbose (kg/h) | 16,1 | 0 | 16,1 |

Les effluents extraits de l'installation sont identifiés dans le tableau 2.

**TABLEAU 2**

| Sorties chromatographiques | Fraction enrichie en L-Iditol (X1) | Fraction enrichie en L-Sorbose (X2) | Eau excédentaire | Total |
|---|---|---|---|---|
| Débit (l/h) | 198 | 261 | 261 | 720 |
| Densité | 1,009 | 1,005 | 1,0 | - |
| Matières sèches (%) | 9,9 | 6,2 | <0,1 | 35,8 |
| Débit massique (kg/h) | 199,8 | 262,3 | | 462,1 |
| Richesse L-Iditol (%) | 99,4 | <0,1 | | - |
| Débit massique en L-Iditol (kg/h) | 19,7 | <0,1 | | 19,7 |
| Teneur L-Sorbose (%) | <0,1 | 98,8 | | - |
| Débit massique L-Sorbose (kg/h) | <0,1 | 16,1 | | 16,1 |

Ce résultat correspond à un taux d'extraction du L-iditol de pratiquement 100%.

La fraction X2 enrichie en L-sorbose a été hydrogénée à l'aide du catalyseur au nickel de RANEY sous une pression d'hydrogène de 45 bars et à une température de 120° C. Elle a fourni un sirop d'une richesse de 54 % en L-iditol et de 46 % en D-sorbitol.

Elle aurait pu être hydrogénée dans les mêmes conditions en présence de L-sorbose nouvellement mis en oeuvre.

Une partie de la fraction X1 titrant comme il a été indiqué plus avant, une richesse de 99,4 % de L-iditol a été concentrée sous vide à une matière sèche de 80% puis elle a été refroidie à une température de 25° C en 24 heures sous agitation lente. La masse cristallisée a été essorée et lavée avec un peu d'éthanol absolu. On a obtenu du L-iditol cristallisé avec un rendement de 55% en un seul jet. Après séchage en lit fluidisé, on a obtenu des cristaux titrant moins de 1% d'humidité et contenant un peu d'éthanol à l'état de traces. La pureté de ce L-iditol était de plus de 99,9 %.

Une autre partie de cette fraction X1 a été concentrée à une matière sèche de 70% et on a placé 700 g de ce sirop dans un ballon d'évaporateur de laboratoire de marque ROTAVAPOR. On y a ajouté 0,1% sur matières sèches d'acide sulfurique à 98% et l'on a maintenu ce ballon sous vide dans un bain d'huile chauffée à 150° C.

On a suivi le déroulement de la réaction par des prélèvements effectués toutes les 30 minutes durant deux heures et demie. On a analysé la teneur du milieu réactionnel en iditol, iditans et isoidide par HPLC. Les résultats figurent dans le tableau 3 qui suit.

**TABLEAU 3**

| Temps (h) | % iditol | % iditans | % isiodide | % autres |
|---|---|---|---|---|
| 0 | 100 | 0 | 0 | 0 |
| 0,5 | 97,7 | 2,3 | 0 | 0 |
| 1,0 | 61,1 | 28,3 | 10,6 | 0 |
| 1,5 | 22,5 | 32,3 | 45,2 | 0 |
| 2,0 | 0,9 | 5,3 | 92,9 | 0,9 |
| 2,5 | 0 | 0 | 98,8 | 1,2 |

Au terme de la réaction on a dilué le milieu dans son poids d'eau et on a traité la solution avec 2% de noir durant 1 heure à 70° C. On a ensuite procédé à la déminéralisation du sirop d'isoidide sur lit mélangé de résines échangeuses d'ions. On a ensuite fait cristalliser l'isoidide en concentrant le sirop à une matière sèche de 66,5% (estimation réfractométrique) puis en le refroidissant sous agitation lente à raison de 2°C/h. On a ensuite essoré et lavé à l'acétone les cristaux obtenus.

On a obtenu un produit titrant 10,9% d'humidité et 0,4% d'iditans. On n'a pas détecté d'isosorbide ni d'isomannide.

## Revendications

1. Procédé de préparation et de purification de L-iditol, **caractérisé par le fait qu**'un sirop d'un mélange de L-iditol et de L-sorbose est soumis à un traitement chromatographique conduisant à l'obtention d'au moins deux fractions dont l'une est fortement enrichie en L-iditol (fraction X1) et dont l'autre est fortement enrichie en L-sorbose (fraction X2).

2. Procédé selon la revendication 1, **caractérisé par le fait que** la fraction X1 enrichie en L-iditol présente une richesse en L-iditol de 80 à 99,9%, ces pourcentages étant exprimés en poids de matières sèches et le complément à 100% étant constitué essentiellement de L-sorbose.

3. Procédé selon la revendication 2, **caractérisé par le fait que** la fraction X1 enrichie en L-iditol présente une richesse en L-iditol de 90 à 99,5%.

4. Procédé selon la revendication 3, **caractérisé par le fait que** la fraction X1 enrichie en L-iditol présente une richesse en L-iditol de 95 à 99,5%.

5. Procédé selon l'une quelconque des revendications précédentes, **caractérisé par le fait que** le mélange de L-iditol et de L-sorbose est obtenu par fermentation d'une solution de D-sorbitol et de L-iditol à l'aide d'un microorganisme producteur de déshydrogénases du genre *Acetobacter* ou *Gluconobacter,* propre à transformer le D-sorbitol en L-sorbose.

6. Procédé selon la revendication 5, **caractérisé par le fait que** la solution de D-sorbitol et de L-iditol est obtenue par hydrogénation catalytique de L-sorbose.

7. Procédé selon l'une quelconque des revendications 1 à 6, **caractérisé par le fait que** la fraction fortement enrichie en L-sorbose (fraction X2) est collectée pour être mélangée au L-sorbose destiné à l'hydrogénation catalytique.

8. Procédé selon l'une des revendications 1 à 7, **caractérisé par le fait que** le traitement chromatographique est effectué de manière continue.

9. Procédé selon la revendication 8, **caractérisé par le fait que** le traitement chromatographique est effectué à l'aide de résines cationiques fortes chargées en ions alcalins ou alcalino-terreux.

10. Procédé selon la revendication 9, **caractérisé par le fait que** les ions alcalino-terreux sont le calcium.

11. Procédé selon l'une quelconque des revendications qui précèdent, **caractérisé par le fait que** la fraction X1 est utilisée pour obtenir par concentration des cristaux de L-iditol.

12. Procédé selon les revendications 1 à 10, **caractérisé par le fait que** la fraction X1 est utilisée, sans en cristalliser le L-iditol, pour la fabrication de l'isoidide.

## Claims

1. Method for preparing and purifying L-iditol, **characterized in that** a syrup of a mixture of L-iditol and of L-sorbose is subjected to a chromatographic treatment resulting in at least two fractions being obtained, one of which is highly enriched in L-iditol (fraction X1) and the other of which is highly enriched in L-sorbose (fraction X2).

2. Method according to claim 1, **characterized in that** the L-iditol-enriched fraction X1 has an L-iditol richness of 80 to 99.9%, these percentages being expressed as weight % of dry matter, the remainder up to 100 % consisting essentially of L-sorbose.

3. Method according to claim 2, **characterized in that** the L-iditol-enriched fraction X1 has an L-iditol richness of 90 to 99.5%.

4. Method according to Claim 3, **characterized in that** the L-iditol-enriched fraction X1 has an L-iditol richness of 95 to 99.5 %.

5. Method according to any of the preceding claims, **characterized in that** the mixture of L-iditol and of L-sorbose is obtained by fermentation of a solution of D-sorbitol and of L-iditol using a microorganism that produces dehydrogenases of the Acetobacter of Gluconobacter genus, able to convert D-sorbitol to L-sorbose.

6. Method according to claim 5, **characterized in that** the solution of D-sorbitol and of L-iditol is obtained by catalytic hydrogenation of L-sorbose.

7. Method according to any of Claims 1 to 6, **characterized in that** the fraction highly enriched in L-sorbose (fraction X2) is collected so as to be mixed with the L-sorbose intended for the catalytic hydrogenation.

8. Method according to one of Claims 1 to 7, **characterized in that** the chromatographic treatment is carried out continuously.

9. Method according to Claim 8, **characterized in that** the chromatographic treatment is carried out using strong cationic resins loaded with alkali metal or alkaline earth metal ions.

10. Method according to Claim 9, **characterized in that** the alkaline earth metal ions are calcium.

11. Method according to any one of the preceding claims, **characterized in that** the fraction X1 is used to obtain, by concentration, L-iditol crystals.

12. Method according to any of claims 1 to 10, **characterized in that** the fraction X1 is used, without crystallizing the L-iditol therein, for the production of isoidide.

## Patentansprüche

1. Verfahren zur Herstellung und Reinigung von L-Idit, **dadurch gekennzeichnet, dass** ein Sirup eines Gemisches von L-Idit und L-Sorbose einer chromatographischen Behandlung unterworfen wird, die zur Gewinnung von mindestens zwei Fraktionen führt, von denen eine stark an L-Idit angereichert ist (Fraktion X1) und die andere stark an L-Sorbose angereichert ist (Fraktion X2).

2. Verfahren nach Anspruch 1, **dadurch gekennzeichnet, dass** die an L-Idit angereicherte Fraktion X1 einen Gehalt an L-Idit von 80 bis 99,9% aufweist, wobei diese Prozentangaben bezogen auf das Gewicht der Trockensubstanzen ausgedrückt sind, und die Ergänzung zu 100% im Wesentlichen aus L-Sorbose besteht.

3. Verfahren nach Anspruch 2, **dadurch gekennzeichnet, dass** die an L-Idit angereicherte Fraktion X1 einen Gehalt an L-Idit von 90 bis 99,5% aufweist.

4. Verfahren nach Anspruch 3, **dadurch gekennzeichnet, dass** die an L-Idit angereicherte Fraktion X1 einen Gehalt an L-Idit von 95 bis 99,5% aufweist.

5. Verfahren nach einem der vorhergehenden Ansprüche, **dadurch gekennzeichnet, dass** das Gemisch aus L-Idit und L-Sorbose durch Fermentation einer Lösung von D-Sorbit und L-Idit mithilfe eines Dehydrogenasen produzierenden Mikroorganismus der Gattung Acetobacter oder *Glucono*bacter erhalten wird, der zur Umwandlung von D-Sorbit in L-Sorbose geeignet ist.

6. Verfahren nach Anspruch 5, **dadurch gekennzeichnet, dass** die Lösung von D-Sorbit und L-Idit durch katalytische Hydrierung von L-Sorbose erhalten wird.

7. Verfahren nach einem der Ansprüche 1 bis 6, **dadurch gekennzeichnet, dass** die stark an L-Sorbose angereicherte Fraktion (Fraktion X2) gesammelt wird, um sie mit L-Sorbose zu mischen, die zur katalytischen Hydrierung bestimmt ist.

8. Verfahren nach einem der Ansprüche 1 bis 7, **dadurch gekennzeichnet, dass** die chromatographische Behandlung kontinuierlich durchgeführt wird.

9. Verfahren nach Anspruch 8, **dadurch gekennzeichnet, dass** die chromatographische Behandlung mithilfe kationischer Harze durchgeführt wird, die stark mit Alkali- oder Erdalkaliionen geladen sind.

10. Verfahren nach Anspruch 9, **dadurch gekennzeichnet, dass** es sich bei den Erdalkaliionen um Calcium handelt.

11. Verfahren nach einem der vorhergehenden Ansprüche, **dadurch gekennzeichnet, dass** die Fraktion X1 zur Gewinnung von L-Idit-Kristallen durch Aufkonzentrierung verwendet wird.

12. Verfahren nach den Ansprüchen 1 bis 10, **dadurch gekennzeichnet, dass** die Fraktion X1, ohne L-Idit darin zu kristallisieren, zur Herstellung von Isoidid verwendet wird.
